# EUROPEAN PATENT APPLICATION

(11) **EP 4 108 764 A1**
(43) Date of publication of application: **28.12.2022**
(21) Application number: 21181551.9
(22) Date of filing: 24.06.2021
(51) Int. Cl.: C12N 5/0784, C12N 5/0786, A61K 31/74, A61K 31/75, C08L 71/00

(54) **METHOD FOR ACTIVATING DENDRITIC CELLS**

(71) Applicant: Freie Universität Berlin, 14195 Berlin (DE)
(72) Inventor: WEINHART, Heidemarie, 12435 Berlin (DE); STÖBENER, Daniel, 12163 Berlin (DE); PEISER, Matthias, 14195 Berlin (DE)
(74) Representative: Maikowski & Ninnemann Patentanwälte Partnerschaft mbB

(57) **Abstract**

The present invention relates to a method for activating dendritic cells in vitro, comprising the following steps: a) providing a substrate, wherein a surface of the substrate comprises at least one poly(glycidyl ether) derivative according to general formula (I); b) contacting the substrate with a dendritic cell in an isosmotic aqueous solution or buffer.

## Description

The present invention relates to a method for activating dendritic cells in vitro according to the preamble of claim 1, to the use of the substrate for activating dendritic cells in vitro according to claim 9, and to medical uses of such a substrate according to claims 11 to 13.

The pivotal role of the immune system is the defense against pathogens entering the human body. However, immune cells also play an essential role in the process of tissue regeneration. [1,2] In addition to these findings, the role of biomaterial-activated immune cells in tissue remodeling has been described indicating that immune cells are an auspicious tool in regenerative medicine. [3]

Today, the human immune system, which comprises an innate and an adaptive part, is regarded as a finely tuned interplay of different cell subsets, which all originate from hematopoietic precursor cells located in the bone marrow. Immature dendritic cells (DCs) constitute the link between innate and adaptive immune system and play therefore a crucial role in the development of the adaptive immune system as mediator of both immunity and tolerance. [4]

After activation to differentiated DCs via contact to pathogens and phagocytosis, DCs process and present individual antigens on their surface and transfer this information to T cells in the lymph nodes. To instruct specific T cells, DCs take up foreign antigens from the blood, the skin, the gastro intestinal tract and the respiratory tract. The cells process the pathogens during maturation and migrate them to local lymph nodes to present naive T cell receptor-bearing antigens as short peptide fragments on major histocompatibility complex (MHC) molecules. After antigen uptake, matured DCs upregulate cell surface molecules, such as CD40, CD80, CD86, CD54 and HLA-DR. Because of their function as the central switch of both the innate and adaptive immune system, differentiated and maturated DCs as well as subsequent T cell subpopulations are intensively studied in clinical research and applied science, especially for the development of new testing systems for risk assessment of pharmaceuticals and chemicals. Furthermore, DCs are used in recent approaches for tumor therapy, curing autoimmune diseases and in alternative *in vitro* methods for the replacement of animal experiments to, for example, test sensitization and immune toxicity. Just recently, the central role of DCs in immunoengineering, in tissue repair and in tissue remodeling was discovered and proposed. [5,6]

It is an object of the present invention to provide a novel method for artificially activating dendritic cells.

This object is achieved, in an aspect, by a method for activating dendritic cells in vitro comprising the steps explained in the following.

First, a substrate is provided. In this context, a surface of the substrate comprises at least one poly(glycidyl ether) derivative according to general formula (I):

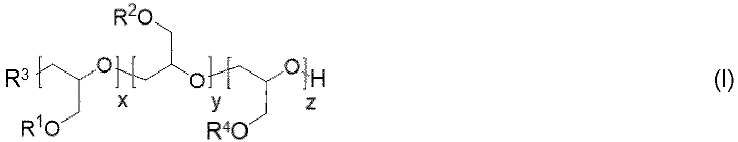

In this context,
- R¹ and R²: denote: independently from each other -H, -CH₃, -CH₂CH₃, -CHCH₂, -CH₂CH₂CH₃, -CH(CH₃)₂, -CH₂CHCH₂, -CH₂CCH, -CH₂CH₂CH₂CH₃, -C(CH₃)₃, -CH₂CH(CH₂CH₃)CH₂CH₂CH₂CH₃, -CH₂(CH₂)₆CH₃, -CH₂(CH₂)₈CH₃, -C₆H₅, -CH₂C₆H₅, -C₆H₄CH₃, or -C₆H₄OCH₃,
- R³: denotes: -H, -Br, -CI, -O-C₁-C₂₀-alkyl, or -O-C₆-C₂₀-aryl,
- R⁴: denotes: a photo-reactive compound according to any of general formulae (II) to (V), wherein the photo-reactive compound is linked to the oxygen atom next to residue R⁴ directly or via a linker molecule: 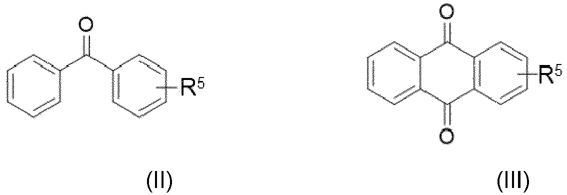 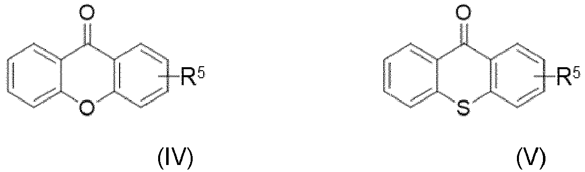 wherein R⁵ is any of the following residues covalently bound to a carbon atom of the structures having general formulae (II) to (V): -H, 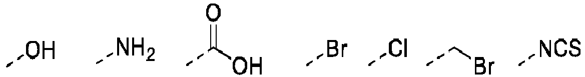 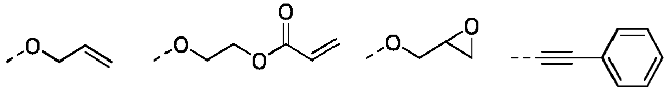 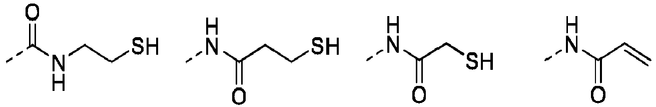 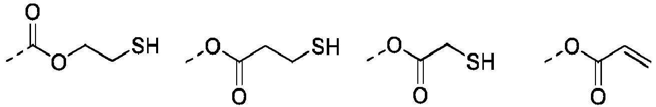 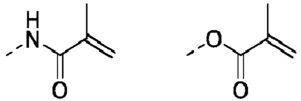 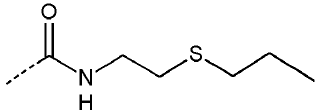
- x: is an integer lying in range of from 0 to 1000,
- y: is an integer lying in range of from 1 to 1000, and
- z: is an integer lying in range of from 1 to 100.

Afterwards, the substrate is contacted with a dendritic cell in an isosmotic aqueous solution or buffer. Such an isosmotic aqueous solution or buffer has the same total osmotic pressure or osmolality as a body fluid, in particular as an intracellular fluid, blood or a blood component such as blood serum or blood plasma, of a human or animal organism, in particular of a human. Typically, the osmolality of such an isosmotic aqueous solution or buffer lies in a range of from 275 to 325 mOsm/kg, in particular of from 280 to 320 mOsm/kg, in particular of from 285 to 315 mOsm/kg, in particular of from 290 to 310 mOsm/kg, in particular of from 295 to 305 mOsm/kg. Due to this contact, the dendritic cell is activated. This activation can also be denoted as "positive" activation. It can be biologically distinguished from an alarm activation (i.e., an activation due to a specific danger for the dendritic cell). The achieved activation results in a positive immunomodulatory effect and/or in a positive tissue repair effect of the activated dendritic cell.

Expressed in other words, a method for the material-induced activation of immune cells utilizing photo-reactive polymers comprising a poly(glycidyl ether) derivative (PGE) is presently described. PGEs can be conveniently immobilized onto common tissue culture substrates, such as polystyrene (PS). Thermoresponsive PGE coatings based on the monomers glycidyl methyl ether (GME) and ethyl glycidyl ether (EGE) have shown to be cell-compatible and have already been used as functional substrates for harvesting confluent 2D cell monolayers with further use in cell sheet engineering applications. [7] However, the potential of PGE coatings for immune cell activation has not yet been reported.

Efforts have been made in the screening of various polymer materials regarding their potential for the activation of DCs. Whereas many biomedical polymer materials only show insignificant activation of DCs [8,9] without an additional DC maturation and activation with lipopolysaccharide (LPS), only few polymer materials, such as chitosan, poly(lactic acid-co-glycolic acid) (PLGA) and some synthetic methacrylate polymers, have shown to activate DCs in a similar manner than the conventional LPS treatment. [10,11,12]

Completely surprisingly, the present inventors found that PGEs trigger the maturation and activation of dendritic cells (DCs). This unanticipated and unprecedented finding makes PGE coatings promising materials in the field of immunoengineering, which is a new area of research in experimental medicine and tissue remodeling.

The chemical structure of the PGE derivative corresponding to general formula (I) is chosen such to achieve a particularly appropriate binding to a mammalian cell. In an embodiment, the dendritic cell is a mammalian cell. In an embodiment, the dendritic cell to be activated forms part of a cell culture.

In an embodiment, the substrate comprises or essentially consists of polystyrene (PS), tissue culture polystyrene (TCPS), polyethylene terephthalate (PET), polyethylene (PE), polypropylene (PP), polycarbonate (PC) or a mixture thereof.

In an embodiment, x is an integer lying in a range of from 50 to 900, in particular of from 100 to 800, in particular of from 200 to 700, in particular of from 300 to 600, in particular of from 400 to 500.

In an embodiment, y is an integer lying in a range of from 50 to 900, in particular of from 100 to 800, in particular of from 200 to 700, in particular of from 300 to 600, in particular of from 400 to 500.

In an embodiment, z is an integer lying in a range of from 5 to 90, in particular of from 10 to 80, in particular of from 20 to 70, in particular of from 30 to 60, in particular of from 40 to 50.

In an embodiment, the poly(glycidyl ether) derivative forms a coating on the substrate, wherein the coating fulfils at least one of the following criteria.

According to a first criterion, a dry thickness of the coating lies in range of from 2 nm to 50 nm, in particular of from 3 nm to 45 nm, in particular of from 4 nm to 40 nm, in particular of from 5 nm to 35 nm, in particular of from 6 nm to 30 nm, in particular of from 7 nm to 25 nm, in particular of from 8 nm to 20 nm, in particular of from 9 nm to 15 nm, in particular of from 10 nm to 13 nm.

According to a second criterion, a static water contact angle of the coating lies in a range of from 65° to 85°, in particular of from 70° to 80°, in particular of from 71° to 79°, in particular of from 72° to 78°, in particular of from 73° to 77°, in particular of from 74° to 76°. In an embodiment, the static water contact angle is measured by applying the so-called sessile drop method at 20 °C with a drop volume of 2 µL. In an embodiment, the static water contact angle is measured according to DIN EN ISO 19403-2.

In an embodiment, static water contact angles (CAs) are measured with an OCA contact angle system (e.g., from DataPhysics Instruments GmbH (Filderstadt, Germany)) and fitted with the software package SCA202 (version 3.12.11) using the sessile drop method. In an embodiment, CAs are determined before and after surface functionalization at 20 °C. In an embodiment, a drop of Milli-Q water (2 µL) is placed onto the respective surface and CAs are then determined with the Young-Laplace model. In an embodiment, CAs are measured for each substrate on at least five different spots of the coating to test for the homogeneity of the sample. In an embodiment, at least six independent substrates (n = 6) are measured to assure reproducibility of the CA measurement.

According to a third criterion, a surface roughness of the coating lies in a range of from 0.5 nm to 10 nm, in particular of from 1 nm to 9 nm, in particular of from 1.5 nm to 8 nm, in particular of from 2 nm to 7 nm, in particular of from 3 nm to 6 nm, in particular of from 4 nm to 5 nm.

The surface roughness as used herein corresponds, in an embodiment, to the root mean squared surface roughness Rq or RMS. It is typically 10 % higher than the arithmetical mean deviation of the assessed profile Ra. In an embodiment, the surface roughness, in particular Rq, is measured according to ISO 4287:1997.

In an embodiment, R¹ is -CH₃ and R² is -CH₂CH₃. Then, the PGE derivative is a copolymer of glycidyl methyl ether (GME) monomers and ethyl glycidyl ether (EGE) monomers.

In an embodiment, a ratio between x and y lies in a range of from 50:50 to 0:100, in particular of from 40:60 to 10:90, in particular of from 30:70 to 20:80. In an embodiment, a ratio between z and a sum of x and y (i.e., z:(x+y)) lies in a range of from 1:100 to 5:100, in particular of from 1.5:100 to 4:100, in particular of from 2:100 to 3:100. Thus, in this embodiment, repeating units or monomers carrying residue R⁴ make up between 1 % and 5 % of all other repeating units or monomers of the PGE derivate.

In an embodiment, the repeating units carrying residue R⁴ are statistically distributed over the poly(glycidyl ether) derivative.

In another embodiment, the repeating units carrying residue R⁴ are present in the poly(glycidyl ether) derivative as at least one block.

In an embodiment, the poly(glycidyl ether) derivative is present on the surface of the substrate in form of a gel or a brush. Brushes are structures in which the individual molecules are more regularly arranged than in gels and that are typically slightly more hydrophobic than gels when having the same poly(glycidyl ether) constitution. Additionally, brushes are less deformable than gels. Thus, upon applying pressure onto a coating, the thickness of a gel coating can be reduced to a higher extent than the thickness of a brush coating. A coating in form of a brush structure has typically an overall smaller thickness (e.g., a maximum thickness being smaller than 5 to 10 nm) than a coating in form of a gel structure (e.g., a maximum thickness being higher than 5 to 10 nm). Surprisingly, it was found that a gel structure of the coating is able to activate dendritic cells both for tissue repair purposes and for immune response purposes. In contrast, a brush structure of the coating was primarily able to activate dendritic cells for tissue repair purposes, but not for immune response purposes. Thus, depending on the desired kind of activation of the dendritic cell, the structure of the coating can be adapted.

In an embodiment, the dendritic cell is chosen from the group consisting of monocyte-derived dendritic cells, myeloid dendritic cells, plasmacytoid dendritic cells, Langerhans cells, Kupffer cells, and subpopulations of the before-mentioned dendritic cells.

In an aspect, the present invention relates to the use of a substrate for activating dendritic cells in vitro, wherein a surface of the substrate comprises at least one poly(glycidyl ether) derivative according to general formula (I)

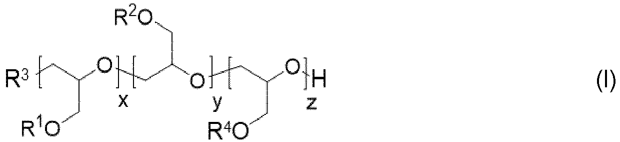

In this context,
- R¹ and R²: denote: independently from each other -H, -CH₃, -CH₂CH₃, -CHCH₂, -CH₂CH₂CH₃, -CH(CH₃)₂, -CH₂CHCH₂, -CH₂CCH, -CH₂CH₂CH₂CH₃, -C(CH₃)₃, -CH₂CH(CH₂CH₃)CH₂CH₂CH₂CH₃, -CH₂(CH₂)₆CH₃, -CH₂(CH₂)₈CH₃, -C₆H₅, -CH₂C₆H₅, -C₆H₄CH₃, or -C₆H₄OCH₃,
- R³: denotes: -H, -Br, -CI, -O-C₁-C₂₀-alkyl, or -O-C₆-C₂₀-aryl,
- R⁴: denotes: a photo-reactive compound according to any of general formulae (II) to (V), wherein the photo-reactive compound is linked to the oxygen atom next to residue R⁴ directly or via a linker molecule: 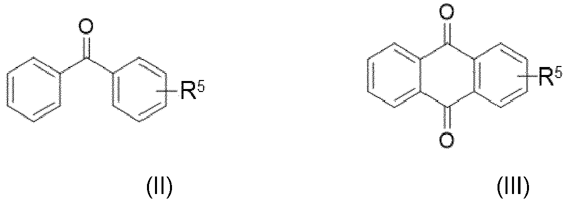 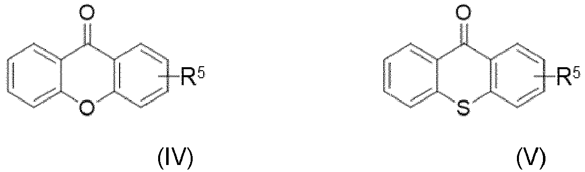 wherein R⁵ is any of the following residues covalently bound to a carbon atom of the structures having general formulae (II) to (V): -H, 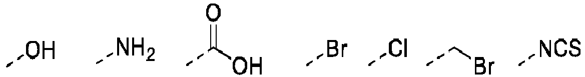 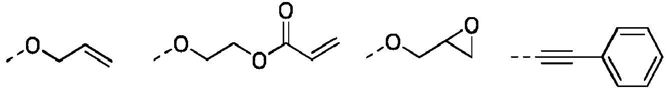 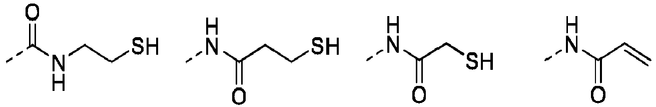 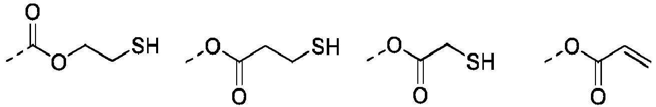 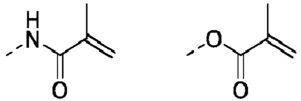 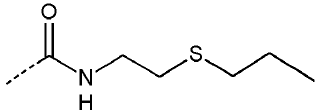
- x: is an integer lying in range of from 0 to 1000,
- y: is an integer lying in range of from 1 to 1000, and
- z: is an integer lying in range of from 1 to 100.

In an embodiment, the activated dendritic cells are used as growth factor source.

In an aspect, the present invention relates to the medical use of a substrate, namely to its use as a medicament or in therapy. In this context, a surface of the substrate comprises at least one poly(glycidyl ether) derivative corresponding to general formula (I) according to the preceding explanations. For the sake of brevity, the concrete structure will not be repeated again here. The substrate can well be used as implant in a human or animal body. Likewise, it can be applied to the human or animal body in form of a patch. In an embodiment, the animal is a mammal.

In an embodiment, the substrate is used in regeneration of skin, heart, cartilage, joint, liver and/or brain. Such organ regeneration is particularly helpful as possible therapy after a disease like cardiac infarction, arthritis, hepatitis or stroke. In the same or another embodiment, the substrate is used in wound healing. Since the substrate activates dendritic cells, it is able to trigger or enhance tissue repair mechanisms of an organism. Therefore, these uses are particularly appropriate applications of the substrate.

In an embodiment, the substrate is used for enhancing a subject's natural tissue repair mechanism or for enhancing or regulating a subject's natural immune response. Regulation of the immune system can be appropriate, e.g., in the case of adverse effects such as a cytokine storm caused by an exuberant immune response, as has been observed, e.g., in the respiratory syndrome associated with COVID-19 disease. As explained above, if the coating has the form of a brush, it is particularly appropriate to selectively activate dendritic cells for enhancing the immune response of the subject. If the coating is applied to the substrate in form of a gel, the substrate will be able to not only enhance the subject's immune response, but also the subject's tissue repair mechanisms.

In an aspect, the invention relates to a method for activating dendritic cells in vivo. This method is generally carried out as the in vitro method explained above, but performed in vivo.

In an aspect, the present invention relates to a medical method for treating a patient in need thereof, wherein the method comprises the steps explained in the following. First, dendritic cells are extracted from the patient, i.e., a human or animal body, in particular a human or mammalian body. Afterwards, the extracted dendritic cells are activated in vitro according to a method as explained above. Afterwards, the activated dendritic cells are re-implanted to the same patient in their activated state. Thus, this method employs an activation of or autologous cells so that an immune therapy or tissue repair therapy making use of this method is particularly compatible for the subject and results in a higher success quote than comparable prior art methods.

All embodiments of the described methods can be combined in any desired way and can be transferred either individually or in any arbitrary combination to the respective other methods, and to the medical and non-medical uses. Likewise, all embodiments of the medical and non-medical uses can be combined in any desired way and can be transferred either individually or in any arbitrary combination to the respective other uses and to the described methods.

Further details of aspects of the present invention will be explained in the following with respect to exemplary embodiments and accompanying Figures. In the Figures:
- Figure 1A: shows the general chemical structure of poly(glycidyl ether) (PGE) copolymers and schematic structures of PGE brush and PGE gel coatings;
- Figure 1B: shows the dry thickness of PGE coatings on PS coated silicon wafer model substrates determined by spectroscopic ellipsometry;
- Figure 1C: shows static water contact angle (CA) of PGE coatings on PS coated silicon wafer model substrates determined by goniometry;
- Figure 2A: shows a representative surface roughness profile of a PGE brush 1:3 on PS coated silicon wafer model substrates measured by atomic force microscopy (AFM) in water at standard cell culture temperature of 37 °C;
- Figure 2B: shows a representative surface roughness profile of a PGE gel 1:3 on the same substrate under the same measuring conditions as in Figure 2A;
- Figure 2C: shows a representative surface roughness profile of a PGE gel 1:7 (C) on the same substrate under the same measuring conditions as in Figure 2A;
- Figure 3A: shows a quantitative histogram of the coating deformation of a PGE gel 1:3 and a PGE brush 1:3 coating on PS coated silicon wafer model substrates measured by atomic force microscopy (AFM) in water at standard cell culture temperature of 37 °C;
- Figure 3B: shows a quantitative histogram of the elastic DMT modulus of the PGE gel and the PGE brush coating of Figure 3A under the same measuring conditions as in Figure 3A;
- Figure 3C: shows a quantitative histogram of the adhesive force of the PGE gel and the PGE brush coating of Figure 3A under the same measuring conditions as in Figure 3A;
- Figure 4A: shows dot plots of FACS analyses of DCs generated from primary monocytes in PBMCs of n=5 healthy donors indicating an increase of DC-specific markers by polymers in flow cytometry.
- Figure 4B: shows the mean fluorescence intensity (FI) and percentage of positive cells of one representative donor of the samples of Figure 4A;
- Figure 5A: shows an upregulation of CD86 on the surface of human DCs by gel polymers after blood derived DCs were cultured on different polymer-coated plates;
- Figure 5B: shows an upregulation of CD40 by the same gel polymers as in Figure 5A;
- Figure 5C: shows an upregulation of HLA-DR by the same gel polymers as in Figure 5A;
- Figure 5D: shows an upregulation of CD14 by the same gel polymers as in Figure 5A;
- Figure 6: shows the release of transforming growth factor (TGF)-β1 by DCs cultured on different polymer-coated plates;
- Figure 7: shows the release of epidermal growth factor (EGF) by DCs cultured on different polymer-coated plates.

The Figures will be explained with respect to an exemplary embodiment.

### Polymer Synthesis

Reports on the activation of immune cells, particularly dendritic cells (DCs), through natural or synthetic culture substrate materials are rather scarce. Most notably, chitosan- and PLGA-based culture substrates have shown to promote the differentiation and activation of DCs, whereas most conventional culture materials do not significantly affect DC maturation. As fully synthetic polymer materials, poly(glycidyl ether) (PGE) coatings are non-toxic, biocompatible and have already demonstrated to facilitate the adhesion and proliferation of mammalian as well as primary human cells.

To study their potential for DC activation, the inventors prepared PGE brush and gel coatings on polystyrene (PS) tissue culture substrates using the "grafting-to" method. The monomer-activated anionic ring-opening polymerization (MA-AROP) of the two comonomers glycidyl methyl ether (GME) and ethyl glycidyl ether (EGE) allows for the synthesis of PGEs with different hydrophilicities via the adjustment of the GME:EGE comonomer ratio. As illustrated in Scheme 1, the inventors synthesized two types of photo-reactive PGE terpolymers comprising benzophenone (BP) moieties, which can be utilized to covalently immobilize and/or crosslink PGEs on PS culture substrates via convenient UV irradiation. PGE block copolymers bearing a short BP anchor block were synthesized via the sequential MA-AROP of GME, EGE and the photo-reactive comonomer 4-(2,3-epoxypropoxy) benzophenone (EBP) (Scheme 1a). In contrast, statistical terpolymers bearing BP moieties along the PGE backbone were synthesized via the two-step post-functionalization of allyl-bearing terpolymers composed of GME, EGE and allyl glycidyl ether (AGE) (Scheme 1b).

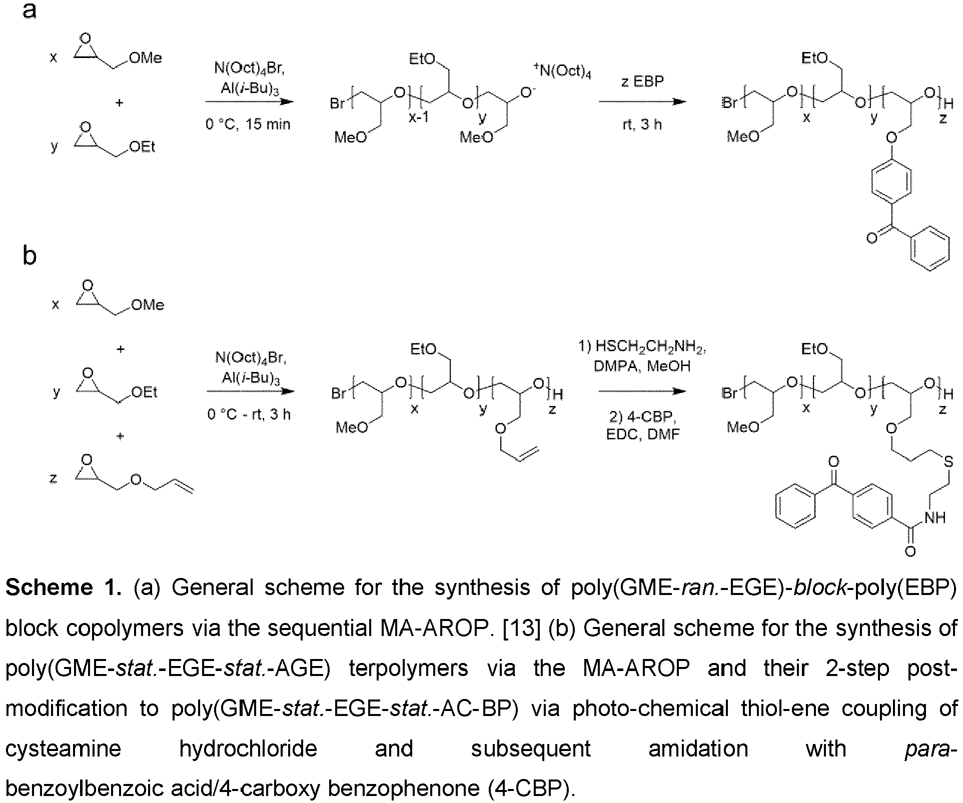

The targeted as well as experimentally determined composition and molecular weight data of all synthesized PGE terpolymers are summarized in Table 1. For the fabrication of brush coatings, PGE block copolymers B1 and B2 with GME:EGE comonomer ratios of 1:1 and 1:3, respectively, and in each case an average of 5 EBP repeating units as well as a number average molecular weight (*Mₙ*) of 30 kDa were targeted (Table 1, entry 1-2). Statistical PGE terpolymers G1, G2 and G3, with GME:EGE comonomer ratios of 1:1, 1:3 and 1:7, respectively, and in each case an average of 1.5 mol-% AGE repeating units as well as an *Mₙ* of 40 kDa were targeted to fabricate hydrogel coatings (Table 1, entry 3-5).

Table 1. Targeted (theoretical) and experimental molecular weight data and comonomer composition of PGE block copolymers B1 and B2 and statistical PGE terpolymers G1, G2 and G3 determined by gel permeation chromatography (GPC) and ¹H-NMR spectroscopy.

| *PGE* | *M_{n,theor}. [kDa]* | *M_{n,GPC} [kDa]* | *PDI_{GPC}* | *GME:EGE (theor.)* | *GME:EGE (NMR)* | *BP units (theor.)* | *BP units (NMR)* |
|---|---|---|---|---|---|---|---|
| ***B1*** | 30 | 28.4 | 1.21 | 1:1 | 1.1:1.0 | 5.0 | 4.2 |
| ***B2*** | 30 | 27.1 | 1.20 | 1:3 | 1.0:2.8 | 5.0 | 4.8 |
| ***G1*** | 40 | 47.5 | 1.19 | 1:1 | 1.0:1.0 | 1.5% | 1.2% |
| ***G2*** | 40 | 45.1 | 1.28 | 1:3 | 1.0:2.8 | 1.5% | 1.2% |
| ***G3*** | 40 | 46.9 | 1.25 | 1:7 | 1.0:6.0 | 1.5% | 1.1% |

As shown in Table 1, the molecular weights of polymers B1-G3 determined by gel permeation chromatography (GPC) are close to the targeted values and all polymers display adequately narrow polydispersity indices (PDI), which indicates the controlled nature of the MA-AROP process. Further, the comonomer compositions determined via ¹H-NMR spectroscopy closely resemble the targeted comonomer ratios. As compared to the aimed at contents, slightly lower experimental yields in terms of BP functionalization can be attributed to the decreased reactivity of the EBP monomer in the sequential MA-AROP of PGE block copolymers (B1-B2) as well as to the incomplete, added up yields during the two-step post-functionalization process of statistical PGE terpolymers (G1-G3). Representative ¹H-NMR spectra of the PGE block copolymers and the statistical PGE terpolymers have also been recorded (not shown).

### Coating and Surface Characterization

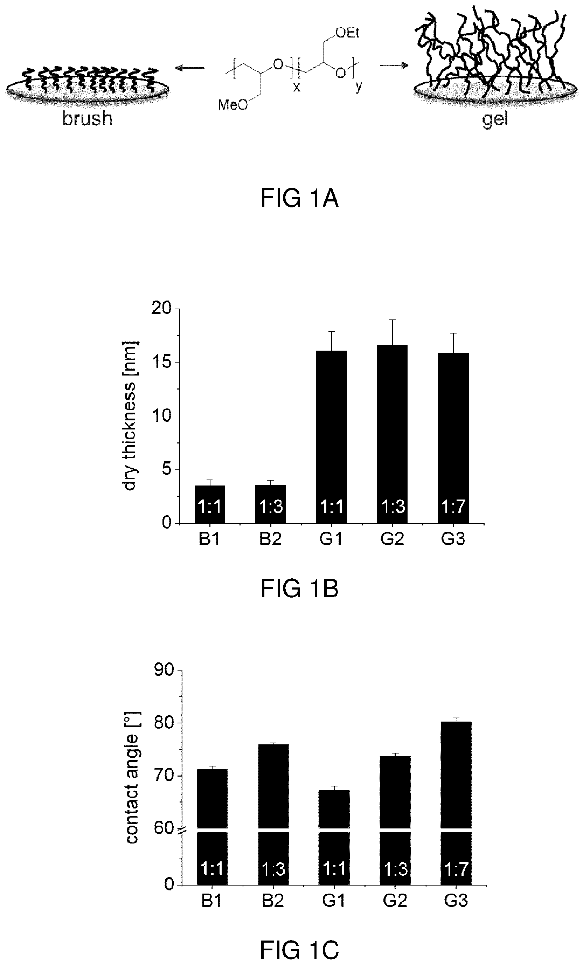

Two different "grafting-to" protocols were utilized to immobilize PGE coatings onto PS culture substrates. PGE brushes were fabricated via the adsorption of PGE block copolymers onto PS substrates from dilute aqueous-ethanolic solution (c_{EtOH} = 32% (B1) and 45% (B2) (v/v)) at a polymer concentration of 62.5 µg mL⁻¹. In general, PS substrates were incubated in PGE solutions for 1 h, washed with water and irradiated with UV light in the dry state to covalently immobilize the self-assembled brushes on the PS surfaces via their EBP anchor block. After extraction of unbound polymer chains with ethanol overnight, stable PGE brush layers were obtained. PGE gels were fabricated by spin coating statistical PGE terpolymers onto PS substrates from ethanolic solution at a polymer concentration of 1% (w/w). The layers were immobilized and crosslinked via UV irradiation and extracted with ethanol for 5 days until the thickness of the coatings adopted steady values. The thickness and wettability of the coatings, which were determined on PS-coated silicon wafer model substrates using spectroscopic ellipsometry (SE) and static water contact angle (CA) measurements, respectively, are summarized in Figure 1.

Figure 1A shows the general chemical structure of poly(glycidyl ether) (PGE) copolymers and schematic structures of PGE brush and PGE gel coatings. Figure 1B shows the dry layer thickness of PGE brush and gel coatings determined by spectroscopic ellipsometry (SE) on PS-coated silicon wafer model substrates. Figure 1C shows the wettability of PGE brush and gel coatings determined by static water contact angle (CA) measurements on PS-coated silicon wafer model substrates.

As shown in Figure 1B, PGE brush coatings B1 and B2 with an average dry thickness of about - 3.5 nm were obtained. Under aqueous conditions, the grafting density of PGE chains (*Mₙ* = 30 kDa) is high enough for the polymers to adopt a brush-like conformation and to fully cover the PS substrate surface. In contrast, with average values of around ∼ 15 nm, the dry thickness values of PGE gels G1, G2 and G3 were significantly higher than for the PGE brushes (Figure 1B). As illustrated in Figure 1C, the static water CAs of the coatings range from about 65 to 80° and reflect the hydrophilicity of the coatings, which was adjusted via the GME:EGE comonomer ratio of the statistical as well as block copolymers. Notably, the wettability of PGE coatings with comparable comonomer compositions is higher for PGE gels, which is reflected in the slightly lower water CAs. This is likely due to the higher swelling capability and water uptake capacity of the significantly thicker PGE gels. Nevertheless, the CAs of the two types of PGE coatings are in the same realm and, in general, CA differences are not necessarily commensurable for coatings with different architectures, such as assembled brushes and gel-like networks.

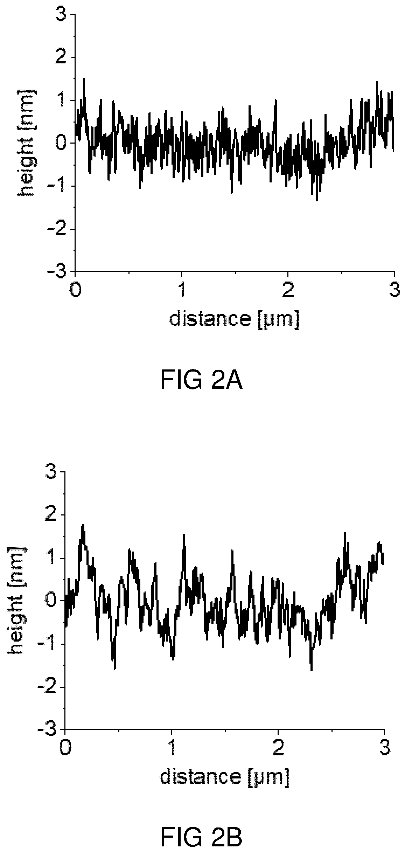

To determine the morphology and mechanical properties of the coated culture substrates, the PGE coatings were characterized by atomic force microscopy (AFM) using quantitative nanomechanical mapping (QNM). Representative topological images of PGE brush and PGE gel coatings on PS-coated silicon wafer model substrates are illustrated in Figures 2A to 2C.

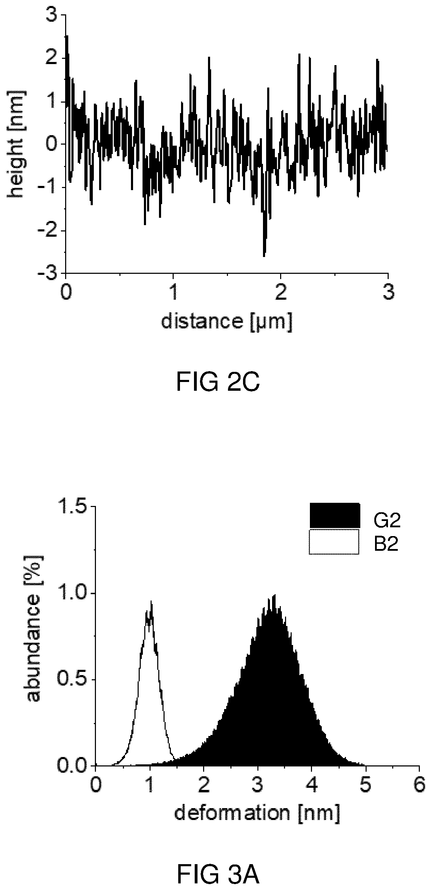

Figure 2A shows a height cross section profile of brush B2 coatings on PS-coated silicon wafer model substrates. Figure 2B shows a comparable height cross section profile for PGE gel G2 coatings, and Figure 2C for PGE gel G3 coatings. All height cross section profiles were measured in water at 37 °C via AFM in quantitative nanomechanical mapping (QNM) mode.

Whereas PGE brushes exhibit a very homogeneous and flat morphology forming a well-ordered layer with a roughness in the range of 1 nm, PGE gels present a slightly rougher and laterally more inhomogeneous surface topology. The slightly higher roughness of PGE gels is due to their higher thickness and crosslinked structure. The more hydrophilic and, hence, swollen nature of PGE gels with a comonomer ratio of 1:3 (G2) (Figure 2B) is further reflected in a morphologically more inhomogeneous, patch-like structure, as compared to PGE gels with a comonomer ratio of 1:7 (G3) (Figure 2C).

It is worth noting that the PS-coated model substrates are very flat compared to the rather rough commercial PS tissue culture substrates. The reason for this is the spin coating process, which was applied for the manufacture of PS-coated silicon wafers. The morphology of the bare PS substrates and PGE-coated silicon wafers as well as common PS culture dishes, respectively, has been further analyzed (not shown).

A comparison between the mechanical properties of the PGE brushes and gels, more precisely, the deformation and elastic modulus of the coatings as well as the adhesive force between the coatings and the AFM tip, is shown in Figures 3A to 3C.

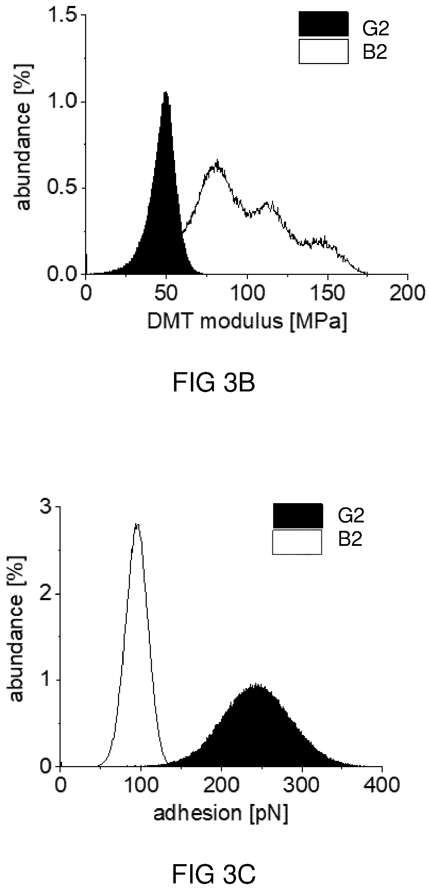

Figure 3A shows representative deformation depth histograms, Figure 3B representative elastic modulus histograms, and Figure 3C representative adhesion histograms of PGE gel G2 and PGE brush B2 coatings measured in water at 37 °C via AFM in quantitative nanomechanical mapping (QNM) mode.

As indicated by the deformation histograms in Figure 3A, PGE gels exhibit a higher deformability than PGE brushes with a similar comonomer composition. Considering that the same indentation force was applied on both surfaces, this is also reflected in a lower elastic modulus of PGE gels, as illustrated in Figure 3B.

Both results on deformation and elastic modulus affirm the brush- and gel-like architecture of the two types of coatings, respectively. Furthermore, the higher adhesive forces between the gel coatings and the AFM tip, which are depicted and compared to PGE brushes in Figure 3C, indicate a more efficient adhesion of the PGE gels to the AFM tip, which is presumably due to its enhanced hydrophobic interactions with the thicker network-like PGE gel coatings.

Summarizing, PGE gels are more deformable (Figure 3A) and exhibit a lower elastic modulus (Figure 3B) than PGE brushes, which is due to the crosslinked, thicker, and, hence, softer structure of PGE gels. The elastic moduli of the coatings are further about one order of magnitude lower than the elastic modulus of standard PS or TCPS culture substrates, making PGE coatings rather soft cell culture substrates. The higher roughness and network-like structure of the gels further increases the adhesive force between the surface and the AFM tip through hydrophobic polymer-tip interactions (Figure 3C).

In general, all investigated brush and gel coatings, respectively, exhibit comparable mechanical properties, which are independent on their comonomer composition within the investigated range. Detailed comparisons between the morphology and the mechanical properties of PGE brush and gel coatings with different comonomer compositions have been established (not shown). In summary, the inventors successfully fabricated PGE-based self-assembled brush as well as crosslinked gel coatings on PS culture substrates. In the following, the potential of these coatings for the activation of DCs will be examined in order to discern their applicability in the field of immunoengineering.

### Activation of dendritic cells (DCs)

In a first series of cell culture experiments, DCs were generated on culture plates coated with different polymers. To evaluate the potential of the synthetic polymer coatings to activate immune cells in general, the crucial immune cell, the DC, which controls the innate immune response and initiates the adaptive immune response, was selected. The range of activation in DCs is known to be measured by the level of cell surface marker expression involved in the antigen presentation processes.

Thus, the costimulatory molecules CD40 and CD86, the maturation marker CD14 for the DC precursor monocyte and the human MHC-II associated molecule HLA-DR were detected. For this purpose, human blood derived DCs were cultured on different polymer-coated plates and analyzed for surface expression of costimulatory CD40 and CD86, HLA-DR and monocyte marker CD14.

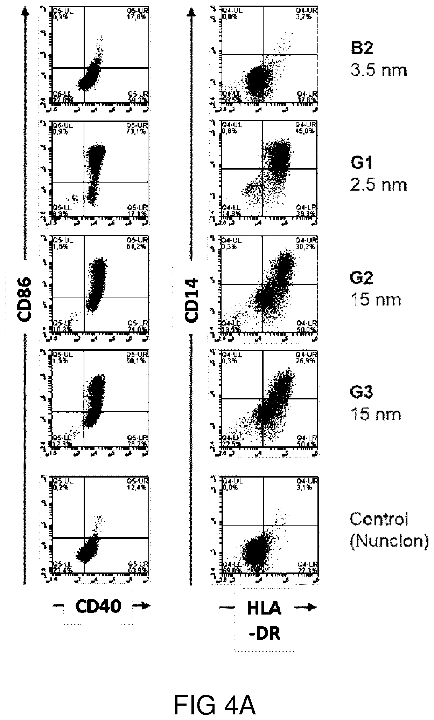

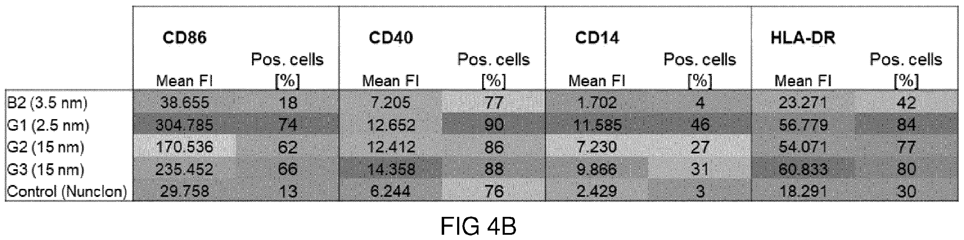

The results are summarized in Figures 4A and 4B. After generation of DCs in presence of GM-CSF and IL-4, according to a scientifically accepted protocol, DCs showed different signs of maturation. After having been cultured on PGE coatings, DCs exhibit morphological characteristics of DCs, such as an enlarged surface area and numerous DC protrusions. Compared to DCs harvested from commercially available cell culture plate controls (Nunclon Delta), activated DCs enhanced cell surface marker expression more pronounced as detected by fluorescence-activated cell scanning (FACS).

Both parameters, fluorescence intensity (FI) and % positive cells, were upregulated in both PGE brushes and gels (Figures 4A and 4B). For CD86, the range of upregulation in fluorescence intensity was higher than for CD40 and HLA-DR. Since the cultured cells resembled a more monocytic phenotype on thin crosslinked gels with a comonomer ratio of 1:1 (G1) and a thickness of 2.5 nm, as evidenced by CD14 values, these conditions were omitted in the next series of experiments.

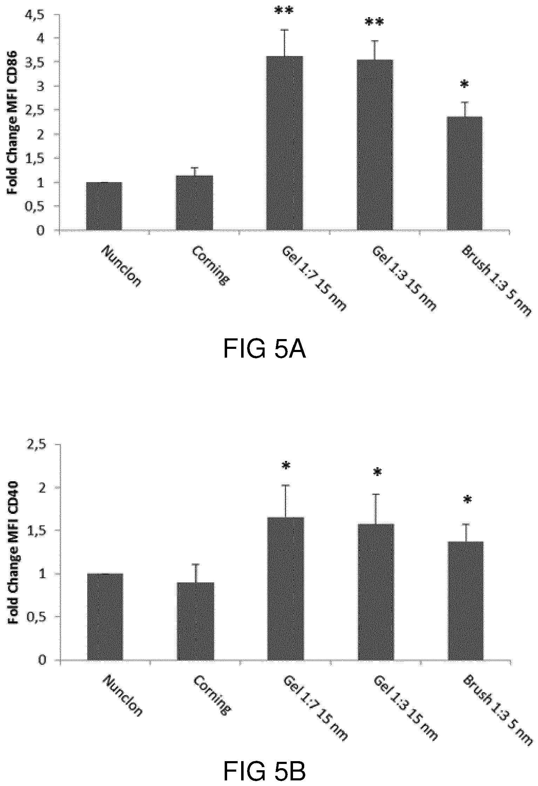

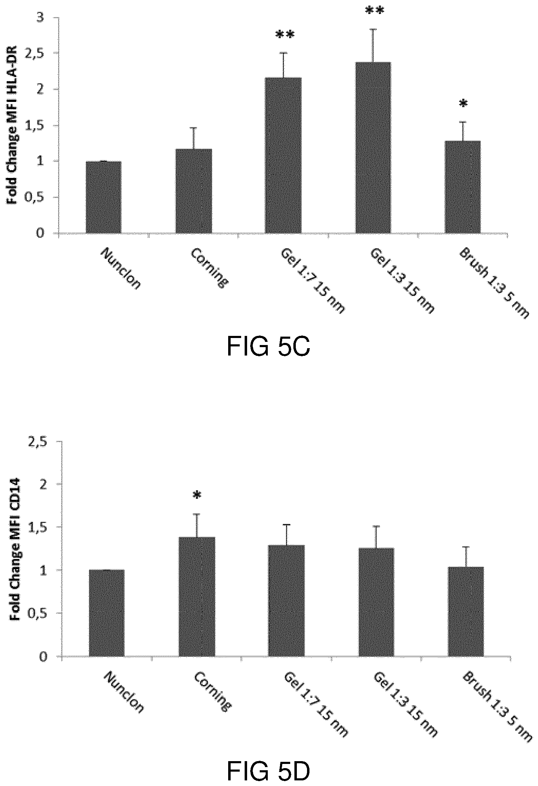

In the next set of cell culture experiments, samples from different human donors were analyzed. The fold change of the mean fluorescence intensity (MFI) revealed that PGE gels increase DC specific markers more than PGE brushes and control plates do. This is summarized in Figures 5A to 5D showing mean fold change in expression (MFI) of costimulatory molecules CD86, CD40 and HLA-DR as well as monocyte marker CD14 of DCs cultured on B2 brush and G2 and G3 gel coatings determined by flow cytometry. Average MFIs of DCs generated from primary monocytes in PBMCs of healthy donors (n = 5) are plotted together with their standard deviations (error bars). Differences to DCs cultured on control substrates ("Nunclon") were analyzed for statistical significance (*, p < 0.05; **, p < 0.01).

Besides "Nunclon", also "Corning" denotes a non-coated surface serving as negative control. The antigen densities of CD86, CD40 and HLA-DR were strongly enhanced by PGE gels. Moreover, the expression of the maturation marker CD14 was not significantly altered compared to control substrates. Hence, all conditions could be regarded as DC generating. Under *in vivo* conditions, the activation of DCs is only achieved by exposure to pathogenic organisms, such as bacteria, protozoa, viruses or fungi, or by exposure to xenobiotica. Numerous publications exist in literature, which deal with the secretion of cytokines, chemokines and growth factors in DCs after exposure to total pathogens, pathogen associated molecular pattern (PAMPS), and chemicals.

However, the present embodiment shows that also synthetic polymers can modulate the expression of immune factors cytokines and chemokines. As only little is known on the release of growth factors by DCs in contact with synthetic polymers, these soluble factors released by surface activated DCs were analyzed in the next series of experiments.

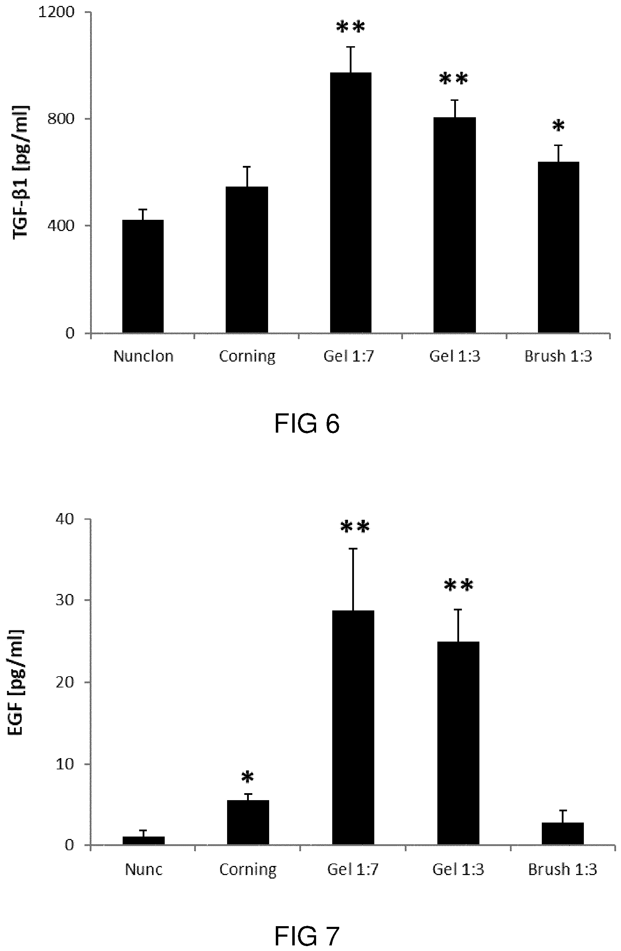

In parallel to conditions identified in the FACS study, it was possible to show that DCs cultured on B2 brush and G2 and G3 gel coatings released transforming growth factor (TGF)-β1 (Figure 6) and epidermal growth factor (EGF) (Figure 7). The proteins in the supernatant of DCs generated from primary monocytes in PBMCs of healthy donors (n = 5) were analyzed by enzyme-linked immunosorbent assay (ELISA) after 4 days of culture and are plotted together with their standard deviations (error bars). Differences in TGF-β1 and EGF expression compared to DCs cultured on control substrates ("Nunclon" or "Nunc") were analyzed for statistical significance (*, p < 0.05; **, p < 0.01).

For TGF-β1 and EGF, the amounts found in the supernatant of DCs were significantly enhanced compared to control plates. Moreover, the amounts of both growth factors were evidently higher when DCs were cultured on PGE gels than on PGE brushes (Figure 6 and 7). These results clearly indicate that DCs are efficiently activated by PGE-based cell culture coatings and that their activation is induced by the synthetic PGE polymer material. There are no reports so far on polyether-based materials, such as PGE coatings, being able to activate DCs. Hence, PGE coatings are very promising materials in the field of immunoengineering.

### Experimental details

### Materials

Glycidyl methyl ether (GME, ≥ 85%), ethyl glycidyl ether (EGE, ≥ 98%), 4-hydroxybenzophenone (4-HBP, ≥ 98%) and cysteamine hydrochloride (Cys-HCI, ≥ 98%) were purchased from TCI GmbH (Eschborn, Germany). Allyl glycidyl ether (AGE, ≥ 99%), tetraethylammonium bromide (N(OCt)₄Br, 98%), triisobutylaluminum (Al(*i*-Bu)₃, 1.0 M in hexanes), 4-benzoylbenzoic acid (4-CBP, 99%), 2,2-dimethoxy-2-phenylacetophenone (DMPA, 99%), *N,N*-dimethylformamide (DMF, 99.8%, anhydrous), benzophenone (BP, 98%), sodium lumps (Na), toluene (99,8%), methanol (99,8%) and ethanol (tech.) were supplied by Merck KGaA/Sigma Aldrich (Darmstadt/Steinheim, Germany). Epichlorohydrin (ECH, 98%) was purchased from abcr GmbH (Karlsruhe, Germany). *N*-(3-dimethylaminopropyl)-*N'-*ethylcarbodiimide hydrochloride (EDC-HCI, ≥ 99%), sodium hydroxide (NaOH, ≥ 98%), sodium sulfate (Na₂SO₄, 99%), molecular sieve (3 Å) and calcium hydride (CaH₂, 93%) were supplied by Carl Roth GmbH + Co. KG (Karlsruhe, Germany). Diethyl ether (Et₂O, 99%) was purchased from VWR Chemicals (Leuven, Belgium). Silicon wafers were supplied by Silchem GmbH (Freiberg, Germany). Toluene was pre-dried via the solvent system MB SPS-800 from M. Braun GmbH (Garching, Germany), refluxed with elemental sodium and a pinch of BP and subsequently distilled on activated molecular sieve directly before use. GME, EGE and AGE were dried over CaH₂, distilled and stored over activated molecular sieve before use. Et₂O and ethanol were distilled before use to remove impurities.

### Methods

¹H and ¹³C NMR spectra were recorded on a Joel ECX at 400 or 500 and 100 or 126 MHz, respectively, and processed with the software MestReNova (version 7.1.2). Chemical shifts were reported in δ (ppm) and referenced to the respective deuterated solvent peak (CDCl₃).

GPC was conducted on an Agilent 1100 Series instrument in THF as the eluent at concentrations of 3.5 mg mL⁻¹ and a flow rate of 1 mL min⁻¹ at 25 °C. Three PLgel mixed-C columns (Agilent, Waldbronn, Germany) with dimensions of 7.5 x 300 mm and a particle size of 5 µm were used in-line with a refractive index detector. Calibration was performed with polystyrene (PS) standards from PSS (Mainz, Germany) and calculation was performed with PSS Win-GPC software. ESI-ToF mass spectral data were obtained on an Agilent 6210 ESI-TOF (Agilent Technologies, Santa Clara, CA, USA) spectrometer at flow rates of 4 mL min⁻¹ and a spray voltage of 4 kV.

Spin-coating was performed using a WS-650-23 spin-coater from Laurell Technologies (North Wales, PA, USA). Silicon wafers (11 x 11 mm) were coated at 3000 rpm for 60 s using 50 µL of a 1% (w/w) solution of PS in toluene. Static water contact angles (CAs) were measured with an OCA contact angle system from DataPhysics Instruments GmbH (Filderstadt, Germany) and fitted with the software package SCA202 (version 3.12.11) using the sessile drop method. CAs were determined before and after surface functionalization at 20 °C. A drop of Milli-Q water (2 µL) was placed onto the respective surface and CAs were determined with the Young-Laplace model. For each substrate, CAs were measured on at least five different spots to test for the homogeneity of the sample and at least six independent substrates (n = 6) to test for reproducibility. The dry layer thickness of the polymer coatings was determined by spectroscopic ellipsometry (SE) at an incident angle of 70° with a SENpro spectroscopic ellipsometer from Sentech Instruments GmbH (Berlin, Germany). The thickness of the SiO₂ layer before spin coating and the additional thickness of the spin-coated PS layers were determined separately using a Cauchy layer for modelling and respective average values of at least five different spots on the surfaces were taken as fixed values for the subsequent modeling of the adsorbed PGE layers. The PGE thickness was measured at wavelengths from 370 nm to 1050 nm and was fitted using a model consisting of the previously measured layers with fixed parameters, a PGE layer with a fixed refractive index of n = 1.45 and air as the surrounding medium. For photo-immobilization, samples with adsorbed PGE layers were irradiated with UV light using a UV-KUB 2 (λ = 365 nm, irradiance = 25 mW cm⁻²) from Kloé (Montpellier, France) for 160 s, which corresponds to a radiant exposure of 4.0 J cm⁻².

### Polymer Synthesis

The sequential monomer-activated anionic ring-opening polymerization (MA-AROP) of poly(GME-*ran*.-EGE)-*block*-poly(EBP) block copolymers B1 and B2 was described in our previous report. [13] The initiator N(Oct)₄Br was melted and dried in a flame dried Schlenk flask (100 mL) under high vacuum at 103 °C and dissolved in dry toluene at room temperature. The solution was cooled to 0 °C with an ice bath and the dry monomers GME and EGE were added. The polymerization was initiated via rapid addition of dry Al(*i*-Bu)₃ activator solution. After stirring for 15 min at 0 °C, the photo-reactive comonomer EBP was added and the reaction was allowed to proceed for 3 h while warming up to room temperature. The reaction was quenched by adding Milli-Q water (∼ 0.5 mL), stirred for 1 h, dried over Na₂SO₄ for 1 h under stirring and filtered. After removing toluene under reduced pressure, the crude polymers were dissolved in Et₂O and residual initiator salts were precipitated by centrifugation at 0 °C. After decanting, Et₂O was evaporated and the block copolymers were dissolved and dialyzed against MeOH for 3 d. PGE block copolymers comprising photo-reactive EBP anchor blocks were protected against light throughout synthesis and workup and stored in stock solutions in ethanol (10 mg mL⁻¹) until further use.

### Poly(GME-ran.-EGE)-block-poly(EBP) (B1):

N(Oct)₄Br = 116 mg (0.21 mmol, 1 eq), GME = 3.00 mL (33.44 mmol, 157.7 eq), EGE = 3.63 mL (33.44 mmol, 157.7 eq), Al(*i*-Bu)₃ = 0.85 mL (0.85 mmol, 4 eq), EBP = 270 mg (1.06 mmol, 5 eq), toluene = 30 mL, yield = 97%; ¹H NMR (500 MHz; CDCl₃): δ(ppm) = 7.71 (m, 4H, BP); 7.52-7.43 (m, 3H, BP); 6.92 (m, 2H, BP); 4.19 (m, 1H, BPOCH₂-); 4.05 (m, 1H, BPOCH₂-); 3.61-3.45 (m, polymer backbone + -OCH₂CH₃); 3.33 (s, -OCH₃); 1.16 (t, - OCH₂CH₃); ¹³C NMR [¹H] (126 MHz; CDCl₃): δ(ppm) = 197.5 (C=O); 165.7 (C(BP)-O-CH₂-polymer backbone); 138.1 115.4 (BP); 78.9-78.6 (-CH₂CH(CH₂OR)O- + BPOCH₂-), 72.8 (-CH₂OCH₃), 70.6 (-CH₂OCH₂CH₃), 70.2-69.6 (-CH₂CH(CH₂OR)O-), 66.7 (-OCH₂CH₃); 59.2 (OCH₃); 15.3 (-OCH₂CH₃); GPC: *Mn* = 28.4 kg mol⁻¹, PDI = 1.21.

### Poly(GME-ran.-EGE)-block-poly(EBP) (B2):

N(Oct)₄Br = 120 mg (0.22 mmol, 1 eq), GME = 1.50 mL (16.72 mmol, 76.1 eq), EGE = 5.45 mL (50.15 mmol, 228.2 eq), Al(*i*-Bu)₃ = 0.88 mL (0.88 mmol, 4 eq), EBP = 279 mg (1.10 mmol, 5 eq), toluene = 30 mL, yield = 98%; ¹H NMR (500 MHz; CDCl₃): δ(ppm) = 7.71 (m, 4H, BP); 7.52-7.43 (m, 3H, BP); 6.92 (m, 2H, BP); 4.19 (m, 1H, BPOCH₂-); 4.05 (m, 1H, BPOCH₂-); 3.61-3.45 (m, polymer backbone + -OCH₂CH₃); 3.33 (s, -OCH₃); 1.16 (t, -OCH₂CH₃); ¹³C NMR [¹H] (126 MHz; CDCl₃): δ(ppm) = 197.5 (C=O); 165.7 (C(BP)-O-CH₂-polymer backbone); 138.1-115.4 (BP); 78.9-78.6 (-CH₂CH(CH₂OR)O- + BPOCH₂-), 72.8 (-CH₂OCH₃), 70.6 (-CH₂OCH₂CH₃), 70.2-69.6 (-CH₂CH(CH₂OR)O-), 66.7 (-OCH₂CH₃); 59.2 (OCH₃); 15.3 (-OCH₂CH₃); GPC: *Mₙ* = 27.1 kg mol⁻¹, PDI = 1.20.

Statistical poly(GME-*stat*.-EGE-*stat*.-AC-BP) terpolymers G1, G2 and G3 were synthesized via the MA-AROP and a subsequent 2-step post-modification via thiol-ene chemistry and amidation. First, allyl-functional poly(GME-*stat*.-EGE-*stat*.-AGE) terpolymers were synthesized according to the following general procedure. The initiator N(Oct)₄Br was melted and dried in a flame dried Schlenk flask (100 mL) under high vacuum at 103 °C and dissolved in dry toluene at room temperature. The solution was cooled to 0 °C with an ice bath and the dry monomers GME, EGE and AGE were added. The polymerization was initiated via rapid addition of dry Al(*i*-Bu)₃ activator solution and stirred for 3 h at 0 °C - rt. The reaction was quenched by adding Milli-Q water (∼ 0.5 mL), stirred for 1 h, dried over Na₂SO₄ for 1 h under stirring and filtered. After removing toluene under reduced pressure, the crude polymers were dissolved in Et₂O and residual initiator salts were precipitated by centrifugation at 0 °C. After decanting, Et₂O was evaporated and the block copolymers were dissolved and dialyzed against MeOH for 3 d. Poly(GME-*stat*.-EGE-*stat*.-AGE) (1a):
N(Oct)₄Br = 174 mg (0.32 mmol, 1 eq), GME = 4.50 mL (50.15 mmol, 157.7 eq), EGE = 5.45 mL (50.15 mmol, 157.7 eq), Al(*i*-Bu)₃ = 1.27 mL (1.27 mmol, 4 eq), AGE = 0.26 mL (2.23 mmol, 7 eq), toluene = 50 mL, yield = 96%; ¹H NMR (500 MHz; CDCl₃): δ(ppm) = 5.87 (m, -OCH₂CHCH₂); 5.27-5.13 (m, -OCH₂CHCH₂); 3.98-3.97 (m, -OCH₂CHCH₂); 3.61-3.47 (m, polymer backbone + -OCH₂CH₃); 3.33 (s, -OCH₃); 1.17 (t, -OCH₂CH₃); GPC: *Mₙ* = 30.1 kg mol⁻¹, PDI = 1.15.

### Poly(GME-stat.-EGE-stat.-AGE) (2a):

N(Oct)₄Br = 184 mg (0.34 mmol, 1 eq), GME = 2.30 mL (25.63 mmol, 79.1 eq), EGE = 8.40 mL (76.90 mmol, 228.2 eq), Al(*i*-Bu)₃ = 1.35 mL (1.35 mmol, 4 eq), AGE = 0.28 mL (2.36 mmol, 7 eq), toluene = 50 mL, yield = 94%; ¹H NMR (500 MHz; CDCl₃): δ(ppm) = 5.87 (m, - OCH₂CHCH₂); 5.27-5.13 (m, -OCH₂CHCH₂); 3.98-3.97 (m, -OCH₂CHCH₂); 3.62-3.47 (m, polymer backbone + -OCH₂CH₃); 3.33 (s, -OCH₃); 1.17 (t, -OCH₂CH₃); GPC: *Mₙ* = 29.2 kg mol⁻¹, PDI = 1.17.

### Poly(GME-stat.-EGE-stat.-AGE) (3a):

N(Oct)₄Br = 174 mg (0.32 mmol, 1 eq), GME = 4.50 mL (50.15 mmol, 157.7 eq), EGE = 5.45 mL (50.15 mmol, 157.7 eq), Al(*i*-Bu)₃ = 1.27 mL (1.27 mmol, 4 eq), AGE = 0.26 mL (2.23 mmol, 7 eq), toluene = 50 mL, yield = 96%; ¹H NMR (500 MHz; CDCl₃): δ(ppm) = 5.87 (m, -OCH₂CHCH₂); 5.27-5.13 (m, -OCH₂CHCH₂); 3.98-3.97 (m, -OCH₂CHCH₂); 3.61-3.47 (m, polymer backbone + -OCH₂CH₃); 3.33 (s, -OCH₃); 1.17 (t, -OCH₂CH₃); GPC: *Mₙ* = 30.1 kg mol⁻¹, PDI = 1.15.

Statistical poly(GME-*stat*.-EGE-*stat*.-AC-BP) terpolymers 1a, 2a and 3a were functionalized with photo-reactive BP units via a two-step post-polymerization protocol according to our previous report. [13] In the first step, the allyl groups were functionalized with cysteamine groups via thio-ene chemistry. In brief, PGE terpolymers 1a, 2a or 3a, Cys-HCI and the photoinitiator DMPA were dissolved in MeOH in a 50 mL reaction vial and purged with Ar for 15 min while protected against light. The reaction mixtures were then irradiated with UV light (Hg lamp, 90 W) for 2 h. The crude products were dialyzed against MeOH for 3 d. After removing the solvent under reduced pressure, the products 1b, 2b and 3b were obtained as highly viscous pale-yellow solids. In the second step, the amine group bearing PGE block copolymers were functionalized with BP groups via amide coupling. In brief, to solutions of 1b, 2b or 3b and 4-CBP in DMF (10 mL) in a 100 mL round bottom flask were added solutions of EDC-HCI in DMF (5 mL). The mixtures were stirred at room temperature for 24 h. The crude product solutions were diluted with MeOH (20 mL) and dialyzed against MeOH for 3 d. After removing the solvent under reduced pressure, G1, G2 and G3 were obtained as highly viscous pale-yellow liquids. The polymers were subsequently stored in stock solutions in ethanol (10 mg mL⁻¹) until further use.

### Poly(GME-stat.-EGE-stat.-AC) (1b):

1a = 2 g (6.7 AGE/chain, 1 eq), Cys-HCI = 254 mg (2.24 mmol, 5 eq), DMPA = 23 mg (0.09 mmol, 0.2 eq), MeOH = 10 mL, conversion (AGE) = 100%, yield = 90%; ¹H NMR (500 MHz; CDCl₃): δ(ppm) = 3.62-3.42 (m, polymer backbone + -OCH₂CH₃); 3.34 (s, -OCH₃); 3.12 (m, -SCH₂CH₂NH₂); 2.94 (m, -SCH₂CH₂NH₂); 2.68 (m, -OCH₂CH₂CH₂S-); 1.85 (m, -OCH₂CH₂CH₂S-); 1.17 (t, -OCH₂CH₃).

### Poly(GME-stat.-EGE-stat.-AC) (2b):

2a = 2 g (7.1 AGE/chain, 1 eq), Cys-HCI = 269 mg (2.37 mmol, 5 eq), DMPA = 24 mg (0.09 mmol, 0.2 eq), MeOH = 10 mL, conversion (AGE) = 100%, yield = 93%; ¹H NMR (500 MHz; CDCl₃): δ(ppm) = 3.61-3.42 (m, polymer backbone + -OCH₂CH₃); 3.33 (s, -OCH₃); 3.12 (m, -SCH₂CH₂NH₂); 2.94 (m, -SCH₂CH₂NH₂); 2.68 (m, -OCH₂CH₂CH₂S-); 1.85 (m, -OCH₂CH₂CH₂S-); 1.17 (t, -OCH₂CH₃).

### Poly(GME-stat.-EGE-stat.-AC) (3b):

3a = 2 g (6.7 AGE/chain, 1 eq), Cys-HCI = 254 mg (2.24 mmol, 5 eq), DMPA = 23 mg (0.09 mmol, 0.2 eq), MeOH = 10 mL, conversion (AGE) = 100%, yield = 90%; ¹H NMR (500 MHz; CDCl₃): δ(ppm) = 3.62-3.42 (m, polymer backbone + -OCH₂CH₃); 3.34 (s, -OCH₃); 3.12 (m, -SCH₂CH₂NH₂); 2.94 (m, -SCH₂CH₂NH₂); 2.68 (m, -OCH₂CH₂CH₂S-); 1.85 (m, -OCH₂CH₂CH₂S-); 1.17 (t, -OCH₂CH₃).

### Poly(GME-stat.-EGE-stat.-AC-BP) (G1):

1b = 1.5 g (5.6 Cys-HCI/chain, 1 eq), 4-CBP = 318 mg (1.41 mmol, 5 eq), EDC-HCI = 270 mg (1.41 mmol, 5 eq), DMF = 15 mL, conversion (Cys-HCI) = 91%, yield = 96%; ¹H NMR (500 MHz; CDCl₃): δ(ppm) = 7.91 (m, 2H, BP); 7.79-7.75 (m, 4H, BP); 7.58 (m, 1H, BP); 7.46 (m, 2H, BP); 3.61-3.42 (m, polymer backbone + -OCH₂CH₃); 3.33 (s, -OCH₃); 2.77 (m, -SCH₂CH₂NHCOBP); 2.62 (m, -OCH₂CH₂CH₂S-); 1.82 (m, -OCH₂CH₂CH₂S-); 1.16 (t, - OCH₂CH₃); GPC: Mn = 29800 g mol⁻¹, PDI = 1.20.

### Poly(GME-stat.-EGE-stat.-AC-BP) (G2):

2b = 1.5 g (5.5 Cys-HCI/chain, 1 eq), 4-CBP = 312 mg (1.38 mmol, 5 eq), EDC-HCI = 265 mg (1.38 mmol, 5 eq), DMF = 15 mL, conversion (Cys-HCI) = 96%, yield = 97%; ¹H NMR (500 MHz; CDCl₃): δ(ppm) = 7.91 (m, 2H, BP); 7.79-7.75 (m, 4H, BP); 7.58 (m, 1H, BP); 7.46 (m, 2H, BP); 3.61-3.42 (m, polymer backbone + -OCH₂CH₃); 3.33 (s, -OCH₃); 2.77 (m, -SCH₂CH₂NHCOBP); 2.62 (m, -OCH₂CH₂CH₂S-); 1.82 (m, -OCH₂CH₂CH₂S-); 1.16 (t, - OCH₂CH₃); GPC: *Mₙ* = 28100 g mol⁻¹, PDI = 1.18.

### Poly(GME-stat.-EGE-stat.-AC-BP) (G3):

3b = 1.5 g (5.6 Cys-HCI/chain, 1 eq), 4-CBP = 318 mg (1.41 mmol, 5 eq), EDC-HCI = 270 mg (1.41 mmol, 5 eq), DMF = 15 mL, conversion (Cys-HCI) = 91%, yield = 96%; ¹H NMR (500 MHz; CDCl₃): δ(ppm) = 7.91 (m, 2H, BP); 7.79-7.75 (m, 4H, BP); 7.58 (m, 1H, BP); 7.46 (m, 2H, BP); 3.61-3.42 (m, polymer backbone + -OCH₂CH₃); 3.33 (s, -OCH₃); 2.77 (m, -SCH₂CH₂NHCOBP); 2.62 (m, -OCH₂CH₂CH₂S-); 1.82 (m, -OCH₂CH₂CH₂S-); 1.16 (t, - OCH₂CH₃); GPC: *Mₙ* = 29800 g mol⁻¹, PDI = 1.20.

### Surface Preparation and Characterization

Silicon wafers (- 11 x 11 mm) were equipped with thin films (∼ 50 nm) of the PS culture substrate material via spin coating. The native SiO₂ layer on the silicon wafers and the thickness of the substrate material films were determined separately by spectroscopic ellipsometry (SE) prior to coating. PGE brush coatings were fabricated by incubation of the coated silicon wafer substrates in dilute (0.25 mg mL⁻¹) aqueous/ethanolic solutions of the block copolymers for 1 h. The polymer solutions were subsequently discarded and the surfaces briefly (∼ 30 s) immersed in water to remove excess polymer solution. After drying under a stream of N₂, the substrates were irradiated with UV light (LED, λ = 365 nm) to covalently immobilize the physically adsorbed PGE layers via their photo-reactive BP anchor blocks. PGE gel coatings were fabricated by spin coating PS-coated silicon wafers with 1% (w/w) solutions of G1, G2 and G3 in ethanol. The substrates were subsequently irradiated with UV light (LED, λ = 365 nm) to covalently immobilize and crosslink the PGE layers via their photo-reactive BP units. All surfaces were then washed with ethanol to extract non-immobilized PGE chains until the thickness of the coatings was constant (∼ 1-3 d).

The dry thickness of the PGE coatings was measured by SE before and after UV irradiation as well as during extraction with ethanol. The wettability of the obtained PGE coatings was characterized by static water contact angle (CA) measurements at 20 °C. AFM measurements were performed on a Nanoscope MultiMode 8 equipped with a fluid cell and a thermal application (TA) controller from Bruker (Billerica, MA, USA). The morphology and material properties of the PGE coatings were measured in PeakForce QNM (Quantitative Nanomechanical Mapping) mode. Coated silicon wafers were mounted on the AFM head, degassed Milli-Q water was inserted into the liquid cell and the TA controller was set to the desired temperature (37 °C) and equilibrated for at least 10 min prior to each measurement. To obtain high resolution images with reduced sample damage, SNL-10 cantilevers from Bruker (Billerica, MA, USA) with a nominal spring constant of 0.3 N m⁻¹ and a tip radius of 2-12 nm were used, and images were recorded with a loading peak force of 0.5 to 1 nN, 512 points per line and scan rates of 1.0 Hz. Obtained images were analyzed with the Nanoscope analysis software (version 1.4) and processed using 1^{st} order flattening.

### Cell Culture and Characterization

### Ethical Approval

For whole blood samples, approval by the ethics committee of the Charité - Universitätsmedizin Berlin was obtained. Anonymized blood samples were obtained from the German Red Cross blood donation service Berlin with informed written consent from all participants. All studies were in accordance to the Helsinki guidelines. No part of these studies was conducted outside of Germany.

### Preparation of blood derived monocytes and generation of DCs

Peripheral human blood of normal donors was obtained by buffy coats from the German Red Cross blood donation service, Berlin. All studies performed were in adherence to the Helsinki guidelines. By depletion of contaminating cells monocytes were magnetically isolated from PBMC (monocyte isolation kit II, Miltenyi Biotec). Cells were cultured in RPMI 1640 supplemented with 2 mM L-glutamine, 100 IU/ml penicillin, 100 µg/ml streptomycin and 10% (v/v) heat-inactivated FCS (Biochrom) for 6 days with human recombinant GM-CSF (100 ng/ml), IL-4 (10 ng/ml, both R&D Systems).

### Flow cytometry

Cells were incubated with anti-CD86 BB515 (2331), CD40 APC (5C3), HLA-DR PerCPCy5 (G46-6) and CD14 PE (M5E2) for 30 min on ice. After washing, cells were analyzed by FACS Accuri (BD Biosciences) flow cytometer using BD Accuri C6 software.

### ELISA

Culture supernatants were analyzed for TGF-β1 and EGF (DuoSet, R&D Systems). Protein was determined by sandwich ELISA after 48 h in the supernatants of 1x 106 DCs per ml.

### Statistical analysis

Student's two-tailed unpaired t test was used to calculate statistical significance. P values below 0.05 were considered significant.

### List of references cited in the preceding sections

[1] Godwin, J.W.; Pinto, A.R.; Rosenthal, N.A. PNAS, 2013, 110 (23), 9415-9420.
[2] Bosurgi, L.; Cao, Y.G.; Cabeza-Cabrerizo, M.; et al. Science, 2017, 356, 1072-1076.
[3] Hubbell, J.A.; Thomas, S.N.; Swartz, M.A. Nature, 2009, 462, 449-460.
[4] Anderson, D.A.; Murphy, K.M.; Briseño, C.G. Cold Spring Harb. Perspect. Biol., 2017, doi: 10.1101/cshperspect. a028613.
[5] Sadtler, K.; Estrellas, K.; Allen, B.W.; et al. Science, 2016, 352, 366-370.
[6] Sadtler, K.; Singh, A.; Wolf, M.T.; et al. Nature Materials, 2016, doi:10.1038/natrevmats.2016.404
[7] Stöbener, D.D.; Hoppensack, A.; Scholtz, J.; Weinhart, M. Soft Matter, 2018, 14, 8333-8343.
[8] Roch, T.; Kratz, K.; Ma, N.; Lendlein, A. Clin. Hemorheol. Microcirc., 2015, 61, 347-357.
[9] Roch, T.; Kratz, K.; Ma, N.; Lendlein, A. Clin. Hemorheol. Microcirc., 2016, 64, 899-910.
[10] Babensee, J.E.; Paranjpe, A. J. Biomed. Mater. Res. Part A, 2005, 74, 503-510.
[11] Babensee, J.E. Sem. Immunol., 2008, 20, 101-108.
[12] Kou, P.M.; Pallassana, N.; Bowden. R.; Cunningham, B.; Joy, A.; Kohn, J.; Babensee, J.E. Biomaterials, 2012, 33, 1699-1713.
[13] Stöbener, D.D; Weinhart, M. Polymers, 2020, 12, 1899.

## Claims

1. Method for activating dendritic cells in vitro, comprising the following steps:
a) providing a substrate, wherein a surface of the substrate comprises at least one poly(glycidyl ether) derivative according to general formula (I) 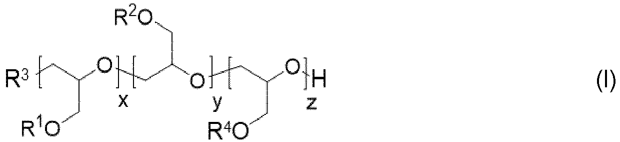 wherein
R¹ and R² = independently from each other -H, -CH₃, -CH₂CH₃, -CHCH₂, -CH₂CH₂CH₃, -CH(CH₃)₂, -CH₂CHCH₂, -CH₂CCH, -CH₂CH₂CH₂CH₃, -C(CH₃)₃, -CH₂CH(CH₂CH₃)CH₂CH₂CH₂CH₃, -CH₂(CH₂)₆CH₃, -CH₂(CH₂)₈CH₃, -C₆H₅, -CH₂C₆H₅, -C₆H₄CH₃, or -C₆H₄OCH₃,
R³ = -H, -Br, -CI, -O-C₁-C₂₀-alkyl, or -O-C₆-C₂₀-aryl,
R⁴ = a photo-reactive compound according to any of general formulae (II) to (V), wherein the photo-reactive compound is linked to the oxygen atom next to residue R⁴ directly or via a linker molecule: 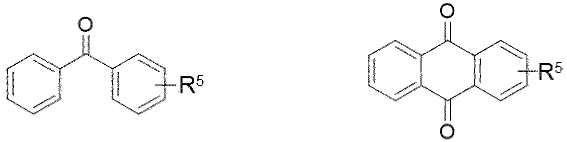 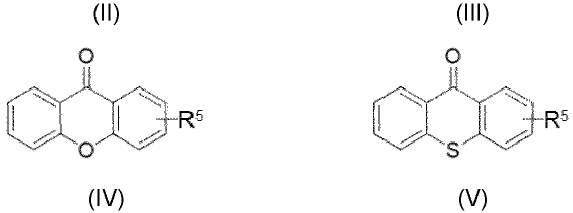 wherein R⁵ is any of the following residues covalently bound to a carbon atom of the structures having general formulae (II) to (V): -H, 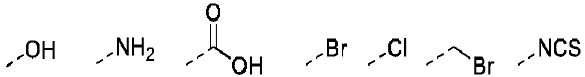 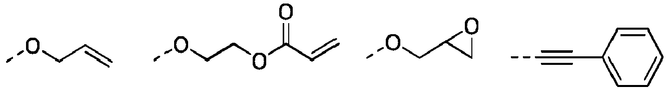 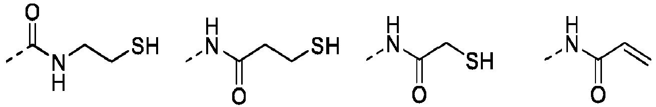 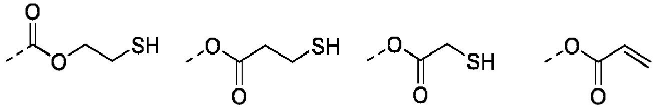 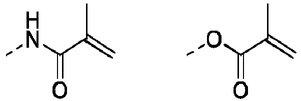 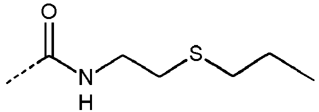
x = 0 to 1000,
y = 1 to 1000, and
z = 1 to 100; and
b) contacting the substrate with a dendritic cell in an isosmotic aqueous solution or buffer.

2. Method according to claim 1, **characterized in that** the poly(glycidyl ether) derivative forms a coating on the substrate, wherein the coating fulfils at least one of the following criteria
a) a dry thickness lying in range of from 2 nm to 50 nm;
b) a static water contact angle lying in a range of from 65° to 85°;
c) a roughness lying in a range of from 0.5 nm to 10 nm.

3. Method according to claim 1 or 2, **characterized in that** R¹ is -CH₃ and R² is -CH₂CH₃.

4. Method according to claim 3, **characterized in that** a ratio between x and y lies in a range of from 50:50 to 0:100, wherein a ratio between z and a sum of x and y lies in a range of from 1:100 to 5:100.

5. Method according to any of the preceding claims, **characterized in that** the repeating units carrying residue R⁴ are statistically distributed over the poly(glycidyl ether) derivative.

6. Method according to any of the preceding claims, **characterized in that** the repeating units carrying residue R⁴ are present in the poly(glycidyl ether) derivative as at least one block.

7. Method according to any of the preceding claims, **characterized in that** the poly(glycidyl ether) derivative is present on the surface of the substrate in form of a gel or a brush.

8. Method according to any of the preceding claims, **characterized in that** the dendritic cell is chosen from the group consisting of monocyte-derived dendritic cells, myeloid dendritic cells, plasmacytoid dendritic cells, Langerhans cells, Kupffer cells, and subpopulations of the before-mentioned dendritic cells.

9. Use of a substrate, wherein a surface of the substrate comprises at least one poly(glycidyl ether) derivative according to general formula (I) 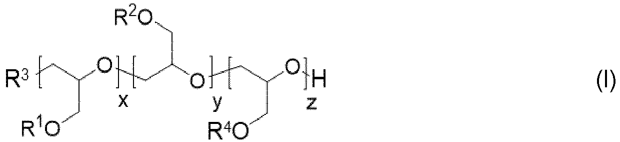 wherein
R¹ and R² = independently from each other -H, -CH₃, -CH₂CH₃, -CHCH₂, -CH₂CH₂CH₃, -CH(CH₃)₂, -CH₂CHCH₂, -CH₂CCH, -CH₂CH₂CH₂CH₃, -C(CH₃)₃, -CH₂CH(CH₂CH₃)CH₂CH₂CH₂CH₃, -CH₂(CH₂)₆CH₃, -CH₂(CH₂)₈CH₃, -C₆H₅, -CH₂C₆H₅, -C₆H₄CH₃, or -C₆H₄OCH₃,
R³ = -H, -Br, -CI, -O-C₁-C₂₀-alkyl, or -O-C₆-C₂₀-aryl,
R⁴ = a photo-reactive compound according to any of general formulae (II) to (V), wherein the photo-reactive compound is linked to the oxygen atom next to residue R⁴ directly or via a linker molecule: 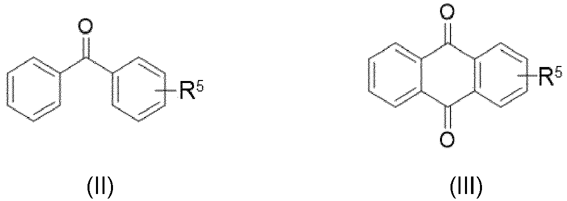 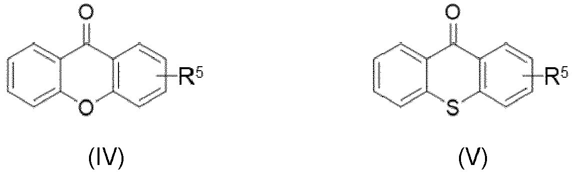 wherein R⁵ is any of the following residues covalently bound to a carbon atom of the structures having general formulae (II) to (V): -H, 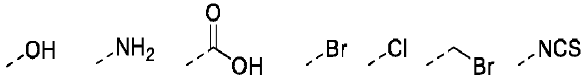 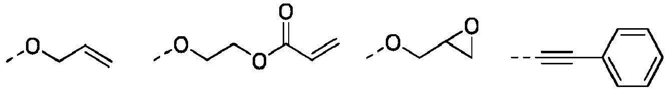 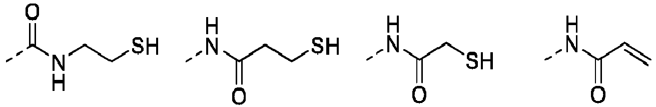 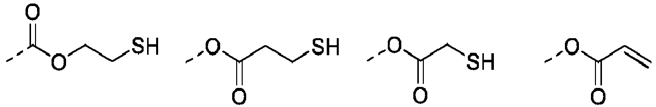 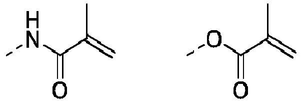 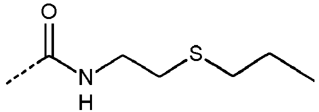
x = 0 to 1000,
y = 1 to 1000, and
z = 1 to 100
for activating dendritic cells in vitro.

10. Use according to claim 9, **characterized in that** the activated dendritic cells are used as growth factor source.

11. Substrate, wherein a surface of the substrate comprises at least one poly(glycidyl ether) derivative according to general formula (I) 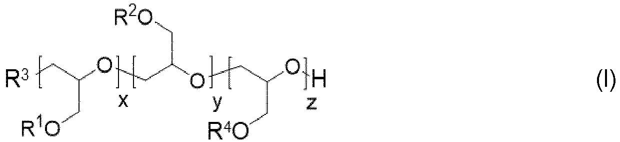 wherein
R¹ and R² = independently from each other -H, -CH₃, -CH₂CH₃, -CHCH₂, -CH₂CH₂CH₃, -CH(CH₃)₂, -CH₂CHCH₂, -CH₂CCH, -CH₂CH₂CH₂CH₃, -C(CH₃)₃, -CH₂CH(CH₂CH₃)CH₂CH₂CH₂CH₃, -CH₂(CH₂)₆CH₃, -CH₂(CH₂)₈CH₃, -C₆H₅, -CH₂C₆H₅, -C₆H₄CH₃, or -C₆H₄OCH₃,
R³ = -H, -Br, -CI, -O-C₁-C₂₀-alkyl, or -O-C₆-C₂₀-aryl,
R⁴ = a photo-reactive compound according to any of general formulae (II) to (V), wherein the photo-reactive compound is linked to the oxygen atom next to residue R⁴ directly or via a linker molecule: 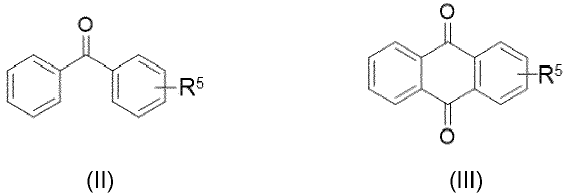 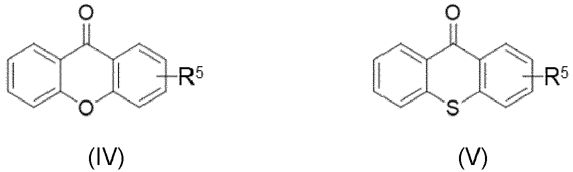 wherein R⁵ is any of the following residues covalently bound to a carbon atom of the structures having general formulae (II) to (V): -H, 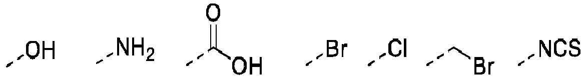 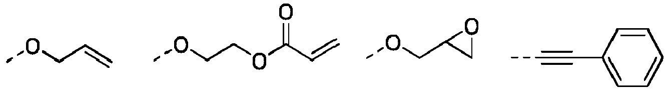 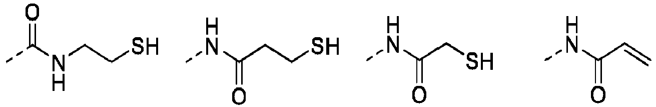 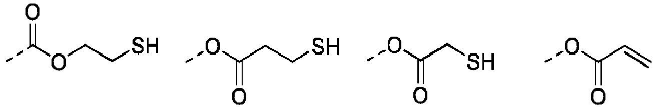 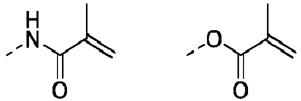 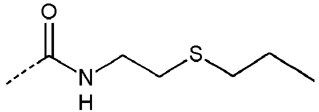
x = 0 to 1000,
y = 1 to 1000, and
z = 1 to 100
for use as a medicament or for use in therapy.

12. Substrate according to claim 11 for use in regeneration of skin, heart, cartilage, joint, liver and/or brain, or for use in wound healing.

13. Substrate according to claim 11 for use in enhancing a subject's natural tissue repair mechanism or for use in enhancing or regulating a subject's natural immune response.

14. Substrate according to claim 11 for use according to any of claims 11 to 13, **characterized in that** the substrate is applied in form of an implant.
